# EUROPEAN PATENT APPLICATION

(11) **EP 4 349 862 A1**
(43) Date of publication of application: **10.04.2024**
(21) Application number: 21942597.2
(22) Date of filing: 27.08.2021
(51) Int. Cl.: C07K 16/24, C12N 15/13, C12P 21/08, A61K 39/395, A61P 37/02, A61P 25/00, A61P 3/10, A61P 1/00, A61P 29/00, A61P 17/06

(54) **ANTI-HUMAN INTERFERON ALPHA RECEPTOR 1 MONOCLONAL ANTIBODY AND APPLICATION THEREOF**

(30) Priority: 27.05.2021 CN 202110586032
(71) Applicant: Qyuns Therapeutics Co., Ltd., Jiangsu 225300 (CN)
(72) Inventor: QIU, Jiwan, Taizhou, Jiangsu 225300 (CN); KONG, Yong, Taizhou, Jiangsu 225300 (CN); CHEN, Wei, Taizhou, Jiangsu 225300 (CN); QIAO, Huaiyao, Taizhou, Jiangsu 225300 (CN); QIU, Zhihua, Taizhou, Jiangsu 225300 (CN); WU, Yiliang, Taizhou, Jiangsu 225300 (CN); CHEN, Tao, Taizhou, Jiangsu 225300 (CN); WU, Meijuan, Taizhou, Jiangsu 225300 (CN)
(74) Representative: Lavoix
(86) International application number: PCT/CN2021/114951
(87) International publication number: WO 2022/247030

(57) **Abstract**

Provided are an anti-human interferon α receptor 1 (IFNAR1) monoclonal antibody and an application thereof. Compared with anti-human IFNAR1 monoclonal antibody Anifrolumab, the anti-human (IFNAR1) monoclonal antibody of the present application has a similar binding affinity to human IFNAR1, and the neutralizing activity thereof at the cellular level is comparable to that of Anifrolumab. In addition, the present application is expected to be used for the prevention and treatment of related diseases.

## Description

### TECHNICAL FIELD

The present application relates to the field of antibody drugs. Specifically, the present application relates to monoclonal antibodies against human interferon alpha receptor 1 (IFNAR1) and uses thereof.

### BACKGROUND TECHNIQUE

Type I interferons (IFNs) (IFNα, IFNβ, IFNω, IFNτ) are a family of structurally related cytokines with antiviral, antitumor, and immunomodulatory effects (Hardy et al., Blood. 97:473, 2001; Cutrone and Langer, J. Biol. Chem. 276:17140, 2001). The human IFNα locus includes two subfamilies. The first subfamily consists of 14 non-alleles and 4 pseudogenes with at least 80% homology. The second subfamily (αII or ω) contains 5 pseudogenes and one functional gene, which shows 70% homology with the IFNα gene (Weissmann and Weber, Prog. Nucl. Acid Res. Mol. Biol., 33:251-300,1986). Isoforms of IFNα have different specific activities, but they share the same biological landscape (Streuli et al., PNAS-USA 78:2848, 1981) and have the same cellular receptor (Agnet M. et al., "Interferon 5", first edition. Gresser pp.1-22, Academic Press, London, 1983).

Interferon beta (IFNβ) is encoded by a single gene with approximately 50% homology to IFNα.

All type I interferons bind to a cell surface receptor (IFNα receptor, IFNAR) composed of two transmembrane proteins, IFNAR1 and IFNAR2. It is reported that IFNAR1 is necessary for high-affinity binding and for the different specificities of the IFNAR complex (Cutrone and Langer, J. Biol. Chem. 276:17140, 2001). Although the functional differences of the various type I interferon subtypes have not yet been determined, it is thought that each of them may interact with different parts of the IFNAR receptor, resulting in possible diverse signaling results (Cook et al. (1996), J. Biol. Chem., 271:13448). In particular, studies using mutant forms of IFNAR1 and IFNAR2 suggest that α and β interferons signal differently through the receptor by interacting differently with the corresponding chains (Lewerenz et al. (1998) J. Mol. Biol., 282:585).

Early functional studies of type I interferons focused on innate defense against viral infection (Haller et al., (1981) J. Exp. Med., 154:199; Lindenmann et al., (1981) Methods Enzymol., 78:181). However, recent studies suggest that type I interferons are potent immunomodulatory cytokines in adaptive immune response. In particular, type I interferons have been shown to favor the differentiation of naive T cells along the Th1 pathway (Brinkmann et al., (1993) J. Exp. Med., 178:1655) to enhance antibody production (Finkelman et al., (1991) J. Exp. Med., 174:1179), and support the functional activity and survival of memory T cells (Santini, et al., (2000) J. Exp. Med., 191:1777; Tough et al., (1996) Science 272:1947).

Many studies suggest that IFNα can enhance the maturation or activation of dendritic cells (DC) (Santini, et al., (2000) J. Exp. Med., 191: 1777; Luft et al., (1988) J. Immunol., 161:1947; Luft et al., (2002) Int. Immunol., 14:367; Radvanyi et al., (1999) Scand. J. Immunol., 50:499). In addition, increased expression of type I interferons has been described in a number of autoimmune diseases (Foulis et al. (1987) Lancet, 2:1423; Hooks et al., (1982) Arthritis Rheum 25:396; Hertzog et al. (1988) Clin. Immunol. immunopathol., 48:192; Hopkins and Meager (1988) Clin. Exp. Immunol. 73:88; Arvin and Miller (1984) Arthritis Rheum. 27:582). The most studied examples of this are insulin-dependent diabetes mellitus (IDDM) (Foulis (1987), supra) and systemic lupus erythematosus (SLE) (Hooks (1982), supra), both of which are associated with the increase of IFNα level, as well as rheumatoid arthritis (RA) (Hertzog (1988), Hopkins and Meager (1988), Arvin and Miller (1984), supra), in which IFNβ may play a more important role.

It has been reported that interferon α administration worsens disease in patients with psoriasis and multiple sclerosis and induces SLE-like syndromes in patients with no previous history of autoimmune disease. It has also been shown that interferon α induces glomerulonephritis in normal mice and accelerates the onset of spontaneous autoimmune disease in NZB/W mice. Furthermore, IFNα treatment has been shown to cause undesirable side effects in some cases, including fever and neurological disturbances. Therefore, there are pathological conditions in which inhibiting type I interferon activity may be beneficial to patients, and drugs that effectively inhibit type I interferon activity are needed.

Anifrolumab, a monoclonal antibody drug targeting IFNAR1 developed by AstraZeneca, is intended to be used in the treatment of systemic lupus erythematosus (clinical phase III), lupus nephritis (clinical phase II) and other diseases.

### SUMMARY OF THE INVENTION

The purpose of the present application is to provide a new anti-human interferon alpha receptor 1 monoclonal antibody, a pharmaceutical composition comprising the monoclonal antibody and the pharmaceutical use of the monoclonal antibody.

That is, the present application comprises:
1. An isolated anti-human interferon alpha receptor 1 monoclonal antibody, comprising three heavy chain complementarity determining regions (CDR-H1, CDR-H2 and CDR-H3) and three light chain complementarity determining regions (CDR-L1 , CDR-L2 and CDR-L3), wherein:
   (a) the amino acid sequence of CDR-H1 (in the present specification, CDR-H1 represents heavy chain CDR1) is shown as SEQ ID NO: 1 (SYYMT);
   (b) the amino acid sequence of CDR-H2 (in the present specification, CDR-H2 represents heavy chain CDR2) is shown as SEQ ID NO: 2 (VINVYGGTYYASWAKG);
   (c) the amino acid sequence of CDR-H3 (in the present specification, CDR-H3 represents heavy chain CDR3) is shown as SEQ ID NO: 3 (EDVAVYMAIDL);
   (d) the amino acid sequence of CDR-L1 (in the present specification, CDR-L1 represents light chain CDR1) is shown as SEQ ID NO: 4 (QASQSISNQLS);
   (e) the amino acid sequence of CDR-L2 (in the present specification, CDR-L2 represents light chain CDR2) is shown as SEQ ID NO: 5 (DASSLAS); and
   (f) the amino acid sequence of CDR-L3 (in the present specification, CDR-L3 represents light chain CDR3) is shown as SEQ ID NO: 6 (LGIYGDGADDGIA).
2. The monoclonal antibody according to item 1, which comprises a heavy chain variable region and a light chain variable region, wherein,
   the amino acid sequence of the heavy chain variable region is shown as SEQ ID NO: 7, and the amino acid sequence is: 
EVQLVESGGGLVQPGGSLRLSCAASGFSLSSYYMTWVRQAPGKGLEWVS
VINVYGGTYYASWAKGRFTISRDNSKNTLYLQMNSLRAEDTAVYYCAREDVA
VYMAIDLWGQGTLVTVSS;
 and
   the amino acid sequence of the light chain variable region is shown as SEQ ID NO: 8, and the amino acid sequence is: 
AIQMTQSPSSLSASVGDRVTITCQASQSISNQLSWYQQKPGKAPKLLIYDA
SSLASGVPSRFSGSRSGTKFTLTISSLQPEDFATYYCLGIYGDGADDGIAFGGGT
KVEIK.
3. An isolated nucleic acid encoding any of the aforementioned monoclonal antibodies.
4. A host cell comprising the nucleic acid according to item 3.
   The nucleic acid may be present on a vector. The vector may be of any type, for example, a recombinant vector such as an expression vector. Any of a variety of host cells can be used. In one embodiment, the host cell is a prokaryotic cell, e.g., *Escherichia coli* (*E. coli*)*.* In another embodiment, the host cell is a eukaryotic cell, e.g., a mammalian cell, such as a Chinese Hamster Ovary (CHO) cell.
5. A method for producing monoclonal antibodies, which comprises culturing the host cell according to item 4 to produce any of the aforementioned monoclonal antibodies.
   The method comprises expressing a recombinant vector encoding the anti-human interferon alpha receptor 1 monoclonal antibody in a suitable host cell, thereby producing the monoclonal antibody. In some embodiments, the method includes culturing a host cell comprising a nucleic acid encoding the anti-human interferon alpha receptor 1 monoclonal antibody, thereby expressing the nucleic acid. The method may further comprise recovering the anti-human interferon alpha receptor 1 monoclonal antibody from a host cell culture or host cell culture medium.
6. A pharmaceutical composition, comprising any of the aforementioned monoclonal antibodies and pharmaceutically acceptable carriers.
   The pharmaceutical composition may further comprise additional therapeutic agents (e.g., different anti-human interferon alpha receptor 1 (IFNAR1) antibodies).
7. The pharmaceutical composition according to item 6, which is used to treat diseases related to interferon-mediated signal transduction.
8. The pharmaceutical composition according to item 7, wherein the diseases related to interferon-mediated signal transduction are: systemic lupus erythematosus, insulin-dependent diabetes mellitus, inflammatory bowel disease, multiple sclerosis, psoriasis, autoimmune thyroiditis, rheumatoid arthritis, glomerulonephritis, HIV infection, AIDS, transplant rejection and/or graft-versus-host disease.
9. Use of any of the aforementioned monoclonal antibodies in the preparation of a medicament for treating diseases related to interferon-mediated signal transduction.
10. The use according to item 9, wherein the diseases related to interferon-mediated signal transduction are: systemic lupus erythematosus, insulin-dependent diabetes mellitus, inflammatory bowel disease, multiple sclerosis, psoriasis, autoimmune thyroiditis, rheumatoid arthritis, glomerulonephritis, HIV infection, AIDS, transplant rejection and/or graft-versus-host disease.
11. A method for treating diseases related to interferon-mediated signal transduction, comprising:
   administering the monoclonal antibody according to any of the aforementioned items or the pharmaceutical composition according to any of the aforementioned items to subjects in need thereof.
12. The method according to item 11, wherein the diseases related to interferon-mediated signal transduction are: systemic lupus erythematosus, insulin-dependent diabetes mellitus, inflammatory bowel disease, multiple sclerosis, psoriasis, autoimmune thyroiditis, rheumatoid arthritis, glomerulonephritis, HIV infection, AIDS, transplant rejection and/or graft-versus-host disease.

### TECHNICAL EFFECT

The present application provides a new anti-human interferon alpha receptor 1 (IFNAR1) monoclonal antibody, comparing with the anti-human interferon alpha receptor 1 monoclonal antibody (Anifrolumab) that has entered clinical phase III, the anti-human IFNAR1 monoclonal antibody of the present application shows comparable affinity for binding to IFNAR1 and comparable neutralizing activity at the cellular level.

The monoclonal antibody of the present application shows a comparable neutralizing activity at the cellular level with Anifrolumab (prepared by expression according to the sequence disclosed in patents), and is expected to show good clinical effects in preventing and treating related diseases.

### DESCRIPTION OF THE DRAWINGS

Fig. 1 is a diagram showing the electrophoresis results of the nucleic acids for constructing the transient expression plasmid of HZD1203-45. Among them, M: Marker; Band 1: PCR product 362VH-Hu6; Band 2: pHZDCH, HindIII/Nhel; Band 3: PCR product 362VK-Hu20; Band 4: pHZDCK, HindIII/BsiWI.
Fig. 2 is a flow chart of transient expression.
Fig. 3 is a electrophoresis detection diagram of QX006N (HZD1203-45-IgG4.1).
Fig. 4 is a graph showing the activity of QX006N (HZD1203-45-IgG4.1) and Anifrolumab in neutralizing human interferon-induced STAT1/2 phosphorylation in HEK Blue IFNα/β cells.
Fig. 5 is a graph showing the activity of QX006N and Anifrolumab in neutralizing human interferon in inhibiting Daudi cell proliferation.
Fig. 6 is a graph showing the activity of QX006N and Anifrolumab in neutralizing human interferon-induced release of CXCL10/IP10 from whole blood.

### DETAILED DESCRIPTION

The scientific and technical terminologies mentioned in the present specification have the same meanings as commonly understood by those skilled in the art. If there is any conflict, the definitions in the present specification shall prevail.

Generally speaking, the terminologies used in the present specification have the following meanings.

As used herein, an "isolated" antibody is one that has been separated from components of its natural environment. In certain embodiments, the antibody is purified to greater than 95% or 99% purity determined by, for example, electrophoresis (e.g., SDS-PAGE isoelectric focusing (IEF), capillary electrophoresis) or chromatography (e.g., ion exchange or reverse HPLC). For a review of methods for evaluating antibody purity, see, e.g., Flatman et al., J. Chromatogr. B848:79-87(2007).

In the present specification, "monoclonal antibody" means an antibody derived from a population of substantially homologous antibodies, i.e., the individual antibodies making up the population are identical and/or bind the same epitope, except for possible variant antibodies (such as comprising naturally occurring mutations or produced during the production process of monoclonal antibody products), such variants usually exist in trace amounts. Unlike polyclonal antibody preparations, which typically include different antibodies targeting different determinants (epitopes), each type of monoclonal antibody in monoclonal antibody preparations targets a single determinant on the antigen. Thus, the modifier "monoclonal" indicates the characterization that the antibody is derived from a substantially homogeneous antibody population and should not be construed as requiring any particular method to produce the antibody. For example, monoclonal antibodies to be used according to the present application can be prepared by a variety of techniques, including but not limited to, hybridoma methods, recombinant DNA methods, phage display methods, and a method using transgenic animals containing all or part of human immunoglobulin loci, such methods and other exemplary methods of preparing monoclonal antibodies are described herein.

In the present specification, "affinity" means the strength of the sum of the non-covalent interactions between a single binding site of a molecule (e.g., an antibody) and its binding partner (e.g., an antigen). Unless otherwise indicated, "binding affinity" as used in the present specification means an intrinsic binding affinity that reflects a 1:1 interaction between members of a binding pair (e.g., antibody and antigen). The affinity of a molecule X for its partner Y can usually be expressed by the equilibrium dissociation constant (KD). Affinity can be measured by common methods known in the art.

In the present specification, Human Interferon alpha/beta Receptor 1 (IFNAR1) represents a membrane protein derived from humans, and the amino acid sequence of its extracellular domain is shown as SEQ ID NO: 9, where, the underlined portion indicates a signal peptide.

SEQ ID NO:9:
MMVVLLGATTLVLVAVAPWVLSAAAGGKNLKSPQKVEVDIIDDNFILRW
NRSDESVGNVTFSFDYQKTGMDNWIKLSGCQNITSTKCNFSSLKLNVYEEIKL
RIRAEKENTSSWYEVDSFTPFRKAQIGPPEVHLEAEDKAIVIHISPGTKDSVMW
ALDGLSFTYSLVIWKNSSGVEERIENIYSRHKIYKLSPETTYCLKVKAALLTSW
KIGVYSPVHCIKTTVENELPPPENIEVSVQNQNYVLKWDYTYANMTFQVQWL
HAFLKRNPGNHLYKWKQIPDCENVKTTQCVFPQNVFQKGIYLLRVQASDGN
NTSFWSEEIKFDTEIQAFLLPPVFNIRSLSDSFHIYIGAPKQSGNTPVIQDYPLIY
EIIFWENTSNAERKIIEKKTDVTVPNLKPLTVYCVKARAHTMDEKLNKSSVFS
DAVCEKTKPGNTSK.

In the present specification, "anti-human interferon alpha receptor 1 monoclonal antibody" means a monoclonal antibody that is capable of binding to human interferon alpha receptor 1 with sufficient affinity such that the monoclonal antibody can be used as a diagnostic and/or therapeutic agent targeting human interferon alpha receptor 1.

The anti-human interferon alpha receptor 1 (IFNAR1) monoclonal antibody of the present application does not bind to proteins unrelated to the target. "Unrelated proteins" here refer to proteins other than the target human interferon alpha receptor 1; "no binding" here means: when the binding capacity of the anti-human interferon alpha receptor 1 (IFNAR1) monoclonal antibody of the application to the target human interferon alpha receptor 1 is taken as 100%, the binding capacity of the anti-human interferon alpha receptor 1 monoclonal antibody of the application to the unrelated protein is less than 10%, for example, 9%, 8%, 7%, 6%, 5%, 4%, 3%, 2%, 1%, or 0.

The anti-human interferon alpha receptor 1 (IFNAR1) monoclonal antibody of the present application may not bind to the interferon alpha receptor 1 of other animal species. "Other animal species" here refers to animal species other than humans, such as marmosets, cynomolgus monkeys, pigs, dogs, rabbits, rats, mice, guinea pigs, etc.; "no binding" here means: when the binding capacity of the anti-human interferon alpha receptor 1 (IFNAR1) monoclonal antibody of the application to the target human interferon alpha receptor 1 is taken as 100%, the binding capacity of the anti-human interferon alpha receptor 1 monoclonal antibody of the application to interferon alpha receptor 1 of other animal species is less than 10%, for example, 9%, 8%, 7%, 6%, 5%, 4%, 3%, 2%, 1%, or 0.

The equilibrium dissociation constant (K_{D}) of the anti-human interferon alpha receptor 1 monoclonal antibody of the present application is ≤1 µM, ≤100 nM, ≤50 nM or ≤40 nM.

Experimental results show that the anti-human interferon alpha receptor 1 (IFNAR1) monoclonal antibody of the present application can specifically bind to the human interferon alpha receptor 1 (IFNAR1).

Comparing with the anti-human IFNAR1 monoclonal antibody (Anifrolumab) that has entered clinical phase III, the anti-human interferon alpha receptor 1 (IFNAR1) monoclonal antibody of the present application is comparable in many biological activities. The above biological activities include, for example, the activity of neutralizing the phosphorylation of STAT1/2 in cells induced by human interferon, the activity of neutralizing the inhibition of Daudi cell proliferation by human interferon, the activity of neutralizing human interferon-induced release of CXCL10/IP10 from whole blood, etc.

In one embodiment, the amino acid sequence of the heavy chain of the anti-human interferon alpha receptor 1 (IFNAR1) monoclonal antibody of the present application is shown as SEQ ID NO: 10; the amino acid sequence of the light chain is shown as SEQ ID NO: 11.
SEQ ID NO: 10: 
EVQLVESGGGLVQPGGSLRLSCAASGFSLSSYYMTWVRQAPGKGLEWVS
VINVYGGTYYASWAKGRFTISRDNSKNTLYLQMNSLRAEDTAVYYCAREDVA
VYMAIDLWGQGTLVTVSSASTKGPSVFPLAPCSRSTSESTAALGCLVKDYFPE
PVTVSWNSGALTSGVHTFPAVLQSSGLYSLSSVVTVPSSSLGTKTYTCNVDHK
PSNTKVDKRVESKYGPPCPPCPAPEFLGGPSVFLFPPKPKDTLMISRTPEVTCV
VVDVSQEDPEVQFNWYVDGVEVHNAKTKPREEQFNSTYRVVSVLTVLHQD
WLNGKEYKCKVSNKGLPSSIEKTISKAKGQPREPQVYTLPPSQEEMTKNQVSL
TCLVKGFYPSDIAVEWESNGQPENNYKTTPPVLDSDGSFFLYSRLTVDKSRWQ
EGNVFSCSVMHEALHNHYTQKSLSLSLGK.
SEQ ID NO: 11:

AIQMTQSPSSLSASVGDRVTITCQASQSISNQLSWYQQKPGKAPKLLIYDA
SSLASGVPSRFSGSRSGTKFTLTISSLQPEDFATYYCLGIYGDGADDGIAFGGGT
KVEIKRTVAAPSVFIFPPSDEQLKSGTASVVCLLNNFYPREAKVQWKVDNALQ
SGNSQESVTEQDSKDSTYSLSSTLTLSKADYEKHKVYACEVTHQGLSSPVTKS
FNRGEC.

Wherein SEQ ID NO: 10 and 11 are both humanized sequences.

In the present specification, "isolated" nucleic acid means a nucleic acid molecule that has been separated from components of its natural environment. Isolated nucleic acids comprise nucleic acid molecules contained in cells that normally contain nucleic acid molecules, but the nucleic acid molecules are present extrachromosomally or at chromosomal locations that are different from their native chromosomal locations.

In the present specification, "isolated nucleic acid encoding an anti-human interferon alpha receptor 1 (IFNAR1) monoclonal antibody" means one or more nucleic acid molecules encoding the heavy chain and light chain of the antibody, including such nucleic acid molecules in a single or separate vectors, as well as such nucleic acid molecules present at one or more locations in the host cell.

In the present specification, "vector" means a nucleic acid molecule capable of amplifying another nucleic acid to which it is linked. The term comprises a vector as a self-replicating nucleic acid structure and a vector that integrates into the genome of a host cell into which it has been introduced. Some vectors can guide the expression of nucleic acids to which they are operably linked. Such vectors are referred to herein as "expression vectors".

In the present specification, "host cell", "host cell line" and "host cell culture" are used interchangeably and refer to cells into which exogenous nucleic acid has been introduced, including progeny of such cells. Host cells include "transformants" and "transformed cells," which include primary transformed cells and progeny derived therefrom (regardless of passage number). The progeny may not be identical in nucleic acid content to the parent cell, but may contain mutations. Mutant progenies with the same function or biological activity screened or selected for the originally transformed cell are included in the present specification.

In the present specification, "pharmaceutical composition" means a preparation that is in a form that enables the biological activity of the active ingredient contained therein to exert its effect, and the composition does not contain additional components that have unacceptable toxicity to the subjects to be administered with the preparation.

In the present specification, "pharmaceutically acceptable carrier" means an ingredient in a pharmaceutical composition other than the active ingredient that is not toxic to the subject. Pharmaceutically acceptable carriers include, but are not limited to buffers, excipients, stabilizers or preservatives.

In the present application, "monoclonal antibodies" are generally human antibodies, which can be prepared using techniques well known to those skilled in the art, for example, human antibodies are generally described in van Dijk, M.A. and van de Winkel, J.G., Curr. Opin. Pharmacol.5:368-374(2001) and Lonberg, N., Curr. Opin. Immunol. 20:450-459(2008).

Antibodies can be prepared by administering immunogens to transgenic animals that have been modified to produce intact human antibodies or intact antibodies with human variable regions in response to antigenic challenge. These animals typically contain part or all of the human immunoglobulin loci, which replace the endogenous immunoglobulin loci, either present extrachromosomally or are randomly integrated into the animal. In such transgenic mice, the endogenous immunoglobulin loci have generally been inactived. For review of methods to obtain human antibodies from transgenic animals, see Lonberg, N., Nat. Biotech. (Nature Biotechnology) 23:1117-1125(2005). See also, for example, the XENOMOUSE™ technology described in U.S. Patent Nos. 6,075,181 and 6,150,584; HUMAB® technology described in US Patent No.5770429; K-MMOUSE® Technology described in US Patent No. 7041870, and VELOCIMOUSE® Technology described in US Patent Application Publication No. US 2007/0061900. The human variable regions of intact antibodies generated from such animals can be further modified, for example, by combination with different human constant regions.

Human antibodies can also be produced by hybridoma-based methods. Human myeloma and mouse-human hybrid myeloma cells for production of human monoclonal antibodies have been described (see, e.g., Kozbor, D., J. Immunol. 133:3001-3005 (1984); Brodeur, B.R. et al., Monoclonal Antibody Production Techniques and Applications, Marcel Dekker, Inc., New York (1987), pp.51-63; and Boerner, P. et al., J. Immunol. 147:86-95 (1991)). Human antibodies produced through human B cell hybridoma technology are also described in Li, J. et al., Proc. Natl. Acad. Sci. USA103:3557-3562(2006). Other methods include those described in, for example, U.S. Patent No. 7,189,826 (which describes the generation of monoclonal human IgM antibodies from hybridoma cell lines), and Ni, Xiandai Mianyixue, 26(4); 265-268 (which describes human-human hybridoma). Human hybridoma technology (Trioma technology) is also described in Vollmers, H.P. and Brandlein, S., Histology and Histopathology 20:927-937 (2005), and Vollmers, H.P. and Brandlein, S., Methods and Findings in Experimental and Clinical Pharmacology 27: 185-191(2005).

Human antibodies can also be generated by isolating variable domain sequences of Fv clones selected from human-derived phage display libraries, and such variable domain sequences can then be combined with the desired human constant domain.

Human antibodies can also be selected based on antibody libraries, i.e., human antibodies can be isolated by screening combinatorial libraries for antibodies with one or more desired activities. For example, a variety of methods for producing phage display libraries and screening such libraries for antibodies possessing desired binding characteristics are known in the art. This method is reviewed, for example, in Hoogenboom, H.R. et al., Methods in Molecular Biology 178:1-37 (2001); and further described in, for example, McCafferty, J. et al., Nature 348:552-554 (1990); Clackson, T. et al., Nature 352:624-628(1991); Marks, J. D. et al., J. Mol. Biol., 222:581-597(1992); Marks, J.D. and Bradbury, A., Methods in Molecular Biology 248:161-175(2003); Sidhu, S.S. et al., J. Mol. Biol.338:299-310(2004); Lee, C.Vet al., J. Mol. Biol. 340:1073-1093(2004); Fellouse, F.A., Proc. Natl. Acad. Sci. USA 101:12467-12472(2004); and Lee, C.V et al., J. Immunol. Methods 284:119-132(2004).

In certain phage display methods, repertoires of VH and VL genes are cloned separately by polymerase chain reaction (PCR) and randomly recombined in a phage library, which is then screened for antigen-binding phage, as described in Winter, G. et al., Ann. Rev. Immunol. 12:433-455(1994). Phages typically display antibody fragments as single chain Fv (scFv) fragments or as Fab fragments. Libraries from immunized sources provide high-affinity antibodies against the immunogen without the need to construct hybridomas. Alternatively, the unimmunized repertoire can be cloned (e.g., from humans) to provide a single source of antibodies against a large number of non-autologus and also autologus antigens without any immunization, as described by Griffiths, A.D. et al., EMBO J, 12:725-734 (1993). Finally, unimmunized libraries can also be generated synthetically by cloning non rearranged V gene segments from stem cells and using PCR primers containing random sequences to encode highly variable CDR3 regions and rearrange them *in vitro,* as described by Hoogenboom, H.R. and Winter, G., J. Mol. Biol. 227:381-388 (1992). Patent publications describing human antibody phage libraries include, for example, U.S. Patent No. 5,750,373 and U.S. Patent Publication Nos. 2005/0079574, 2005/0119455, 2005/0266000, 2007/0117126, 2007/0160598, 2007/0237764, 2007/ 0292936 and 2009/0002360.

The antibody may also be a multispecific antibody, such as a bispecific antibody. Bispecific antibodies are monoclonal antibodies with binding specificities for at least two different sites. Techniques for generating multispecific antibodies include, but are not limited to, recombinant coexpression of two pairs of immunoglobulin heavy chain-light chain with different specificities (see Milstein, C. and Cuello, A.C., Nature 305:537-540 (1983); WO 93/08829; and Traunecker, A. et al., EMBO J. 10:3655-3659 (1991)), and "knob-in-hole" engineering (see, e.g., U.S. Patent No. 5,731,168). Multispecific antibodies can also be produced by engineering electrostatic manipulation effects for generating antibody Fc-heterodimer molecules (WO 2009/089004), crosslinking two or more antibodies or fragments (see e.g., US Patent No. 4676980 and Brennan, M. et al., Science 229:81-83 (1985)), using leucine zippers to generate bispecific antibodies (see e.g., Kostelny, S.A. et al., J. Immunol. 148:1547-1553 (1992)), using the "double antibody" technique for generating bispecific antibody fragments (see e.g., Holliger, P. et al., Proc. Natl. Acad. Sci. USA 90:6444-6448 (1993)), using single chain Fv (scFv) dimers (see e.g., Gruber, M. et al., J. Immunol. 152:5368-5374 (1994)), and preparing three specific antibodies (as described in Tutt, A. et al., J. Immunol. 147:60-69 (1991)).

Monoclonal antibodies described herein also include engineered antibodies with three or more functional antigen-binding sites, including "octopus antibodies" (see, e.g., US 2006/0025576).

Antibodies herein also include multispecific antibodies described in WO 2009/080251, WO 2009/080252, WO2009/080253, WO 2009/080254, WO 2010/112193, WO 2010/115589, WO2010/136172, WO 2010/145792, and WO 201 0/145793, WO 2011/117330, WO 2012/025525, WO 2012/025530, WO 2013/026835, WO2013/026831, WO 2013/164325, or WO 2013/174873.

The monoclonal antibodies described herein may also be antibody variants, for example, it may be desirable to improve the binding affinity and/or other biological properties of the antibodies. Amino acid sequence variants of antibodies can be prepared by introducing appropriate modifications into the nucleotide sequence encoding the antibody, or by peptide synthesis. Such modifications include, for example, deletions, and/or insertions and/or substitutions of residues within the amino acid sequence of the antibody. Any combination of deletions, insertions, and substitutions can be made to obtain the final construct, as long as the final construct possesses the desired characteristics, such as antigen binding. Thus, in certain embodiments, antibody variants are provided with one or more amino acid substitutions. Sites of interest for substitution mutations include HVR and FR, for example, amino acid substitutions can be introduced into the antibody of interest, and screen for products with desired activity, for example, retained/improved antigen binding, reduced immunogenicity, or improved ADCC or CDC.

### Examples

Hereinafter, the present application will be described in more detail through examples. It should be understood that the present application is not limited to these examples.

### Example 1: Preparation of anti-human interferon alpha receptor 1 monoclonal antibody QX006N

Human interferon alpha receptor 1 (IFNAR1) was purchased from Shanghai Novoprotein and used to immunize New Zealand rabbits; B cell cloning technology was used to obtain antigen-binding specific antibody clones, and then the monoclonal antibodies that bind to human IFNAR1 and exhibit human IFNAR1 inhibitory activity were screened out. First, the cell supernatant was tested using Binding ELISA to select clones that bind to human IFNAR1; then the HEK Blue IFN α/β reporter gene cell method was used to detect and pick out clones with inhibitory activity to human IFNAR1. The above immunization and screening processes are entrusted to commercialization companies.

37 clones were selected for recombinant expression and sequenced. After testing, 362# and 1203# had the best cell neutralizing activity, and the sequences of the two clones were very similar. Therefore, 362# was first humanized; when 1203 # was screened and found to have better activity, the 1203# clone was humanized based on the humanization of 362#. NCBI IgBlast was used to perform homology alignment of human IgG germline sequences, IGHV3-66*01 was selected as the heavy chain CDR transplantation template, and the CDR regions (i.e., CDR-H1 (SEQ ID No: 1), CDR-H2 (SEQ ID No: 2), and CDR-H3 (SEQ ID No: 3)) of 1203# clone heavy chain were transplanted into the backbone region of IGHV3-66*01; IGKV1-6*01 was selected as the light chain CDR transplantation template, and the CDR regions (i.e. CDR-L1 (SEQ ID No: 4), CDR-L2 (SEQ ID No: 5) and CDR-L3 (SEQ ID No: 6)) of the 1203# clone light chain were transplanted into the backbone region of IGKV1-6*01; reverse mutation(s) on specific site(s) in the backbone region was performed to obtain the variable region of the monoclonal antibody QX006N of the present application. Finally, the variable region sequence of the humanized heavy chain is shown as SEQ ID NO: 7; the amino acid sequence of the humanized light chain variable region is shown as SEQ ID NO: 8.

The gene for the heavy chain variable region (SEQ ID NO: 7) and the gene for the light chain variable region (SEQ ID NO: 8) were obtained by PCR amplification using the gene sequence of the 362# humanized antibody as a template. The heavy chain expression plasmid pHZDCH was digested with HindIII and NheI; the light chain expression plasmid pHZDCK was digested with HindIII and BsiWI; the PCR amplified genes were inserted into the corresponding expression plasmids using Infusion recombinase to construct the heavy chain expression plasmid pHZDCH-362VH-Hu6 and light chain expression plasmid pHZDCK-362VK-Hu20. During the process of humanization, the gene of 1203# humanized antibody was numbered with 362, and the protein was numbered with 1203.

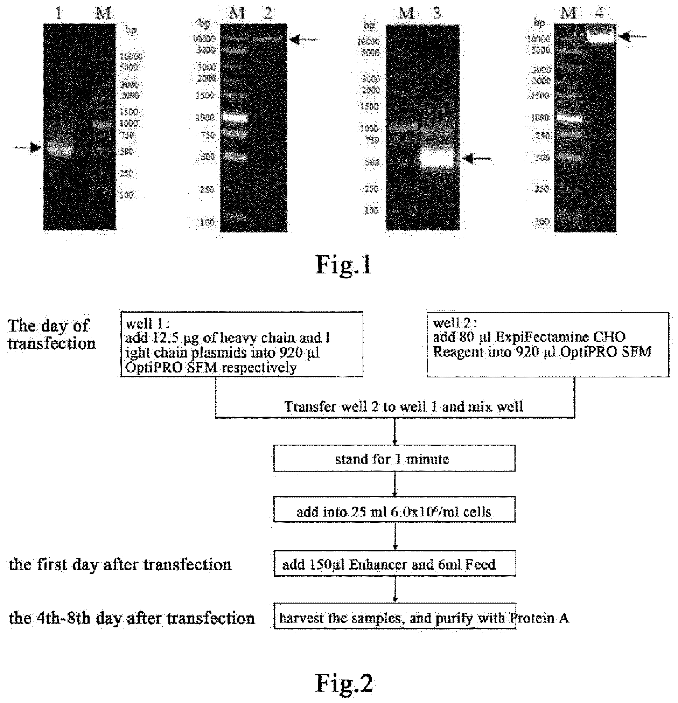

The double digestion results of the plasmid detected by nucleic acid electrophoresis are shown in Fig. 1. According to the results in Fig. 1, the PCR amplification results of the heavy chain variable region and the light chain variable region of the antibody and the results of double-enzyme digestion of the heavy chain and light chain expression plasmids show that the sizes of the heavy chain and light chain plasmids are approximately 10000bp, the light chain variable region is about 447bp, and the heavy chain variable region is about 471bp.

Humanized antibody HZD1203-45 was obtained by humanizing 1203#. In order to reduce the ADCC effect of the antibody, the human IgG1 constant region of the HZD1203-45 heavy chain expression plasmid pHZDCH-362VH-Hu6 was replaced with human IgG4 to obtain the heavy chain expression plasmid pHZDCH-362VH-Hu6-IgG4.1.

The heavy chain expression plasmid pHZDCH-362VH-Hu6-IgG4.1 and the light chain expression plasmid pHZDCK-362VK-Hu20 with the correct sequences were co-transfected into ExpiCHO-S cells. One day before transfection, ExpiCHO-S cells were diluted to 3×10⁶ cells/ml for passage before transfection. On the day of transfection, the cell density was diluted to 6×10⁶ cells/ml, and placed 25 ml of cells in a 125 ml shake flask, waiting for transfection. The transfection and expression process are shown in Fig. 2.

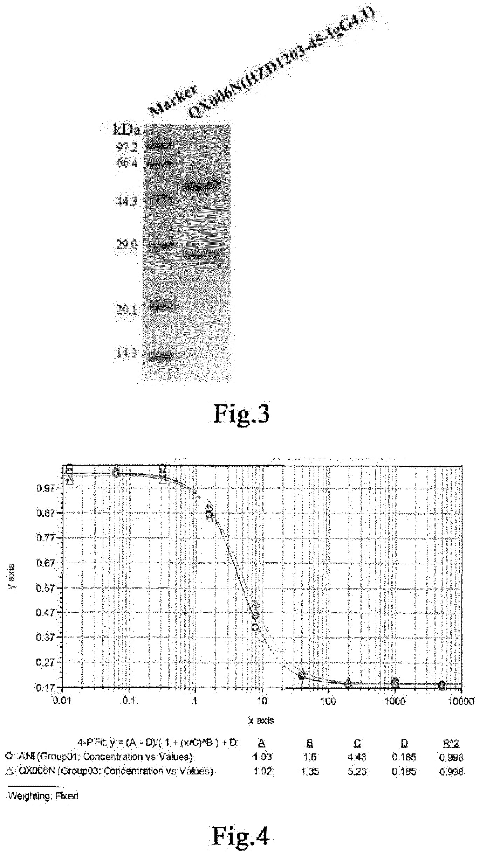

On the 4th-8th day after transfection, the culture supernatant was harvested and purified in one step with ProteinA. The purified antibody was detected by SDS-PAGE electrophoresis and named as QX006N (HZD1203-45-IgG4.1), the amino acid sequence of its heavy chain is shown as SEQ ID NO: 10, and the amino acid sequence of the light chain is shown as SEQ ID NO: 11, and the results of detecting the antibody using protein electrophoresis are shown in Fig. 3. Protein electrophoresis was detected using denaturing reducing gel. The results in Fig. 3 show that there are two bands, the sizes of the two bands are about 50kDa and 25kDa, respectively, which is consistent with the theoretical molecular weight of the heavy chain (48.9kDa) and the light chain (23.4kDa).

### Example 2: Detection of equilibrium dissociation constant (K_{D})

BiacoreT200 was used to detect the affinity between QX006N (HZD1203-45-IgG4.1) and human IFNAR1. All processes were performed at 25°C. A commercial Protein A chip was used to immobilize an appropriate amount of antibody through the capture method so that Rmax was around 50RU and the capture flow rate was 10 µl/min. The antigen was gradient diluted, the flow rate of the instrument was switched to 30 µl/min, flowed through the reference channel and the fixed antibody channel in order of concentration from low to high, and flowed through buffer as the negative control. The chip was regenerated with pH1.5 glycine after each binding and dissociation was completed. The built-in analysis software of the instrument was used to select the 1:1 binding model in the Kinetics option for fitting, and the antibody's binding rate constant *kₐ,* dissociation rate constant *k_{d},* and dissociation equilibrium constant K_{D} values were calculated.

In addition, the affinity of QX006N (HZD1203-45-IgG4.1) was compared with that of Anifrolumab (a monoclonal antibody targeting human IFNAR1 that has entered clinical phase III); the detection method for known antibodies was the same as that of detecting QX006N. The results are shown in Table 1. Wherein Anifrolumab was obtained through constructing an expression plasmid based on the 9D4 sequence provided by patent WO2009100309A2 and transiently transfected into ExpiCHO-S cells.

**Table 1: affinity of antibodies binding to human IFNAR1**

| Name of the samples | *kₐ* (10⁵ M⁻¹S⁻¹) | *k_{d}* (10⁻⁵ S⁻¹) | *K_{D}* (10⁻¹⁰M) |
|---|---|---|---|
| HZD1203-45-IgG4.1 | 3.47 | 3.76 | 1.08 |
| Anifrolumab | 18.67 | 12.40 | 0.67 |

The data in the table were: each sample was tested three times and the average value was calculated.

### Example 3: QX006N and Anifrolumab neutralized human interferon induced STAT1/2 phosphorylation activity in HEK Blue IFNα/β cells

The HEK Blue IFNα/β reporter gene cell line was used to measure the phosphorylation activity of QX006N antagonizing interferon through IFNAR1-mediated intracellular signaling molecule STAT1/2: cells in the culture medium were added into 96 wells at 4×10⁴ cells per well, then incubated overnight at 37°C and 5% CO₂. Serial dilutions of antibody concentrations ranging from 0 to 5 µg/ml were added to the cells, and 0.2 ng/ml of IFNα.2b was added. The cells were then incubated for 24 hours at 37°C and 5% CO₂, collecting the cell culture supernatant and adding 10% QUANTI-Blue^{™} detection reagent to react for 1 hour at 37°C and 5% CO₂; then the OD630nm value was detected a dose-effect curve was drawn, and then the antagonistic activity of the antibody was analyzed, and the dose-effect curve is shown in Fig. 4.

The results in Fig. 4 showed that: QX006N could inhibit the phosphorylation of STAT1/2 in HEK Blue IFNα/β cells induced by interferon; the IC₅₀ of QX006N in inhibiting interferon induced 1/2 phosphorylation activity in HEK Blue IFNα/β cells was 5.23 ng/ml; while the IC₅₀ of Anifrolumab in inhibiting interferon induced 1/2 phosphorylation activity in HEK Blue IFNα/β cells was 4.43ng/ml.

### Example 4: QX006N and Anifrolumab neutralized the activity of human interferon in inhibiting Daudi cell proliferation

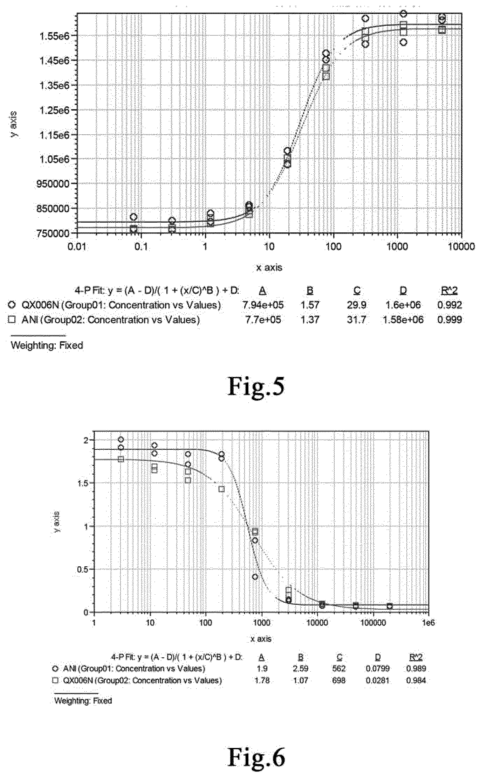

The cell proliferation activity of QX006N antagonizing interferon induced by IFNAR1 was detected using Daudi human lymphoma cell line: cells in the culture medium were added to 96 wells at 4×10⁴ cells per well, and then cultured overnight at 37°C and 5% CO₂. Serial dilutions of antibody concentrations ranging from 0 to 20 µg/ml were added to the cells, and 0.8 ng/ml of IFNα.2b was added. Then, the cells were cultured at 37°C and 5% CO₂ for 72 hours. The cell cultures were collected and CellTiter-Glo was used to detect cell proliferation and draw a dose-effect curve, and then analyze the antagonistic activity of the antibody. The dose-effect curve is shown in Fig. 5.

The results in Fig. 5 showed that: QX006N could inhibit the proliferation of Daudi cells induced by interferon; the EC₅₀ of QX006N in inhibiting the proliferation activity of Daudi cells induced by interferon was 29.9ng/ml; while the EC50 of Anifrolumab in inhibiting the proliferation activity of Daudi cells induced by interferon was 31.7ng/ml.

### Example 5: QX006N and Anifrolumab neutralized activity of human interferon induced release of CXCL10/IP10 in whole blood

The activity of CXCL10/IP10 release of QX006N antagonizing interferon induced by IFNAR1 was detected using human whole blood: whole blood was added to a 96-well plate at 100 µl/well, temporarily stored at 37°C and 5% CO₂; serial dilutions of antibody concentrations ranging from 0 to 40 µg/ml were added to the whole blood, 8 ng/ml of IFNα.2b and 40 ng/ml of TNF-α were also added. Then the cells were cultured at 37°C and 5% CO₂ for 48 hours, the cell culture supernatant was collected, the expression of CXCL10/IP10 in the supernatant was detected using the sandwich ELISA method, a dose-effect curve was drawn, and then the antagonistic activity of the antibody was analyzed. The dose- effect curve is shown in Fig. 6.

The results in Fig. 6 showed that: QX006N could inhibit interferon-induced release of CXCL10/IP10 from whole blood; the IC₅₀ of QX006N in inhibiting interferon induced release of CXCL10/IP10 from whole blood was 698ng/ml; while the IC₅₀ of Anifrolumab in inhibiting interferon induced release of CXCL10/IP10 from whole blood was 562 ng/ml.

## Claims

1. An isolated anti-human interferon alpha receptor 1 monoclonal antibody, comprising three heavy chain complementarity determining regions (CDR-H1, CDR-H2 and CDR-H3) and three light chain complementarity determining regions (CDR-L1, CDR-L2 and CDR-L3), wherein:
(a) the amino acid sequence of CDR-H1 is shown as SEQ ID NO: 1;
(b) the amino acid sequence of CDR-H2 is shown as SEQ ID NO: 2;
(c) the amino acid sequence of CDR-H3 is shown as SEQ ID NO: 3;
(d) the amino acid sequence of CDR-L1 is shown as SEQ ID NO: 4;
(e) the amino acid sequence of CDR-L2 is shown as SEQ ID NO: 5; and
(f) the amino acid sequence of CDR-L3 is shown as SEQ ID NO: 6.

2. The monoclonal antibody according to claim 1, comprising a heavy chain variable region and a light chain variable region, wherein,
the amino acid sequence of the heavy chain variable region is shown as SEQ ID NO: 7; and,
the amino acid sequence of the light chain variable region is shown as SEQ ID NO: 8.

3. An isolated nucleic acid encoding the monoclonal antibody according to claim 1 or 2.

4. A host cell comprising the nucleic acid according to claim 3.

5. A method of producing a monoclonal antibody, which comprises culturing the host cell according to claim 4 to produce the monoclonal antibody according to claim 1 or 2.

6. A pharmaceutical composition, comprising the monoclonal antibody according to claim 1 or 2 and pharmaceutically acceptable carriers.

7. The pharmaceutical composition according to claim 6, which is used to treat diseases related to interferon-mediated signal transduction.

8. The pharmaceutical composition according to claim 7, wherein the diseases related to interferon-mediated signal transduction are: systemic lupus erythematosus, insulin-dependent diabetes mellitus , inflammatory bowel disease, multiple sclerosis, psoriasis, autoimmune thyroiditis, rheumatoid arthritis, glomerulonephritis, HIV infection, AIDS, transplant rejection and/or graft-versus-host disease.

9. Use of the monoclonal antibody according to claim 1 or 2 in the preparation of a medicament for treating diseases related to interferon-mediated signal transduction.

10. The use according to claim 9, wherein the diseases related to interferon-mediated signal transduction are: systemic lupus erythematosus, insulin-dependent diabetes mellitus, inflammatory bowel disease, multiple sclerosis, psoriasis, autoimmune thyroiditis, rheumatoid arthritis, glomerulonephritis, HIV infection, AIDS, transplant rejection and/or graft-versus-host disease.
